# EUROPEAN PATENT APPLICATION

(11) **EP 3 838 254 A1**
(43) Date of publication of application: **23.06.2021**
(21) Application number: 19849484.1
(22) Date of filing: 31.07.2019
(51) Int. Cl.: A61K 8/891, A61K 8/29, A61K 8/34, A61K 8/41, A61Q 1/02, A61Q 17/04

(54) **AQUEOUS DISPERSION COMPOSITION AND USE OF SAME**

(30) Priority: 15.08.2018 JP 2018152803
(71) Applicant: Dow Toray Co., Ltd., Tokyo 140-8617 (JP)
(72) Inventor: KIKUNAGA Sayuri, Ichihara-shi Chiba 299-0108 (JP); PHAN SON THANH, Ichihara-shi Chiba 299-0108 (JP); KANZAKI Yasue, Ichihara-shi Chiba 299-0108 (JP); MIYANO Jun, Tokyo 140-8617 (JP); HORI Seiji, Ichihara-shi Chiba 299-0108 (JP)
(74) Representative: Murgitroyd & Company
(86) International application number: PCT/JP2019/029958
(87) International publication number: WO 2020/036065

(57) **Abstract**

[Object] Provided are an aqueous dispersion composition in which hydrophobic powders are stably and uniformly dispersed in an aqueous phase, which has excellent handling workability, and which can exhibit sufficient water repellency, water resistance, sebum resistance, and the like when compounded in a cosmetic composition or the like; a use of the same (particularly, a material for a cosmetic composition); and a method of producing a cosmetic composition.

[Solving Means] An aqueous dispersion composition which contains at least (A) carboxylic acid-modified silicone in a liquid form at 50°C, (B) a hydrophobic powder, (C) a basic substance, and (D) water; and optionally (E) one or more alcohols selected from polyhydric alcohols and ethyl alcohols; wherein 80% by mass or more of a total composition is formed of the components (A) to (E), and the component (B) is contained in a range of 10% to 70% by mass of the total composition, and the component (A) is contained in a range of 5 to 30 parts by mass with respect to 100 parts by mass of the component (B); a material for a cosmetic composition using the same; or the like.

## Description

### [TECHNICAL FIELD]

The present invention relates to an aqueous dispersion composition of hydrophobic powder and a use of the same, which is particularly useful as a material for a cosmetic composition.

### [BACKGROUND ART]

Inorganic powders, for example, fine particulate zinc oxide or titanium oxide having a particle size of several 10 to 100 nm, are often used as additives or the like in sunscreen cosmetic compositions, outdoor inks, food packaging materials, and the like. When these powders are untreated, there is a risk that a surface is hydrophilic and thus may flow in sweat, rain, or the like. Therefore, particularly in applications of cosmetics and the like, a hydrophobic surface treatment agent such as silane, silicone, and the like, a metal soap, and the like are often used by subjecting a particle surface to a hydrophobization treatment.

Meanwhile, these hydrophobized treatment powders may form an aggregated particle when added to a cosmetic composition or paint because primary particles tend to aggregate, and dispersibility thereof may be insufficient. In particular, for hydrophobic inorganic fine particles having functionality such as fine particulate zinc oxide and fine particulate titanium oxide, from the viewpoint that compounding stability and handling workability for water-based cosmetic compositions and paints can be improved by avoiding the use of ignitable/flammable solvents, it has been proposed to form an aqueous dispersion for the purpose of sufficiently drawing out visible light transparency and ultraviolet light blocking property (Patent Document 1).

However, in these known water-based dispersions, particularly, the uniform dispersibility of hydrophobic inorganic fine particles may be not sufficient, a total composition may be thickened to reduce handling workability, and a cosmetic composition containing the aqueous dispersion may not exhibit sufficient functions such as water repellency, water resistance, sebum resistance, and the like, and therefore, further improvements have been desired. Patent Document 1 does not disclose or suggest a carboxylic acid-modified silicone.

Meanwhile, in Patent Document 2, the present applicant proposes using carboxylic acid-modified silicone under alkaline conditions in order to favorably disperse the hydrophobic powders in an aqueous phase and stabilize the cosmetic composition. However, the literature discloses a method of producing the cosmetic composition itself, in particular, an aqueous composition that does not contain an oil agent, but does not disclose or suggest forming an aqueous dispersion using hydrophobic fine particulate titanium oxide, hydrophobic fine particulate zinc oxide, and the like, as a dispersant using carboxylic acid-modified silicone, and a specific method thereof, and a composition and technical effects thereof.

### [Prior Art Documents]

### [Patent Documents]

Patent Document 1: Japanese Unexamined Patent Application Publication No. 2018-024881
Patent Document 2: Japanese Unexamined Patent Application Publication No. 2015-203026

### [SUMMARY OF THE INVENTION]

### [Problems to be Solved by the Invention]

The present invention has been made to solve the problems described above, and an object of the present invention is to provide an aqueous dispersion composition in which hydrophobic powders are stably and uniformly dispersed in an aqueous phase, and which has excellent handling workability, and can exhibit sufficient water repellency, water resistance, sebum resistance, and the like when compounded in the cosmetic composition or the like.

### [Means for Solving the Problems]

As a result of diligent examination to solve the above problems, the present inventors have reached the present invention by finding that the aforementioned problems can be resolved by an aqueous dispersion composition which may contain at least (A) carboxylic acid-modified silicone in a liquid form at 50°C, (B) a hydrophobic powder, (C) a basic substance, and (D) water; and optionally (E) one or more alcohols selected from polyhydric alcohols and ethyl alcohols, wherein 80% by mass or more of a total composition is formed of the components (A) to (E), and the component (B) is contained in a range of 10% to 70% by mass of the total composition, and the component (A) is contained in a range of 5 to 30 parts by mass with respect to 100 parts by mass of the component (B). In particular, when the carboxylic acid-modified silicone, which is the component (A) described above, is carboxylic acid-modified silicone having a specific structure described below, and is in a liquid form at room temperature (25°C), the aforementioned problems can be very suitably solved.

In other words, the problems of the present invention are solved by:
"[1] An aqueous dispersion composition containing at least
   (A) carboxylic acid-modified silicone in a liquid form at 50°C;
   (B) a hydrophobic powder;
   (C) a basic substance;
   (D) water; and
   optionally, (E) one or more alcohols selected from polyhydric alcohols and ethyl alcohols,
   wherein 80% by mass or more of the total composition is formed of the components (A) to (E),
   the component (B) is contained in a range of 10% to 70% by mass of the total composition, and
   the component (A) is contained in a range of 5 to 30 parts by mass with respect to 100 parts by mass of the component (B).
[2] The aqueous dispersion composition according to [1], wherein 90% by mass or more of the total composition is formed of the components (A) to (E).
[3] The aqueous dispersion composition according to [1] or [2], wherein a content of the component (D) is in a range of 10% to 80% by mass with respect to the total composition.
[4] The aqueous dispersion composition according to any one of [1] to [3], which is substantially free of an oil agent.
[5] The aqueous dispersion composition according to any one of [1] to [4], wherein the (A) carboxylic acid-modified silicone is represented by the following structural formula (1): 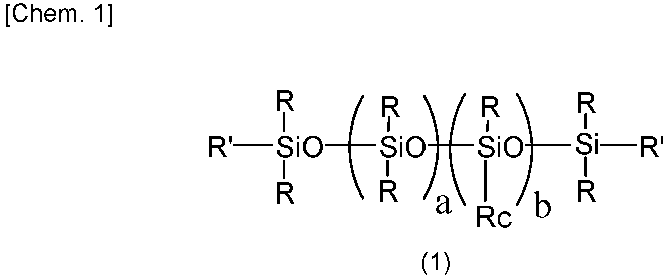 (wherein:
   Rc represents a carboxyl group-containing organic group represented by a general formula: -R¹-(OR²)p-(O)w-R³-COOH, (R¹ represents a linear or branched alkylene group having 2 to 22 carbon atoms, R² represents a linear or branched alkylene group having 2 to 4 carbon atoms, R³ represents a bond (-) or a linear or branched alkylene group having 1 to 22 carbon atoms, p represents a number from 0 to 200, and w represents a number of 0 or 1),
   R represents the same or different alkyl or alkoxy group, having 1 to 22 carbon atoms, or phenyl group,
   R' is Rc or R, and
   a and b are 0 or positive numbers, respectively, a + b is a number in a range of 0 to 30, and when b is 0, at least one of R' is Rc).
[6] The aqueous dispersion composition according to [5], wherein in the structural formula (1), the (A) carboxylic acid-modified silicone is carboxylic acid-modified silicone which is in a liquid form at room temperature (25°C) in which a + b is a number in a range of 2 to 20, and a/b is in a range of 0.3 to 3.0.
[7] The aqueous dispersion composition according to any one of [1] to [6], wherein a pH of the total composition is in a range of 6.5 to 14.0.
[8] The aqueous dispersion composition according to any one of [1] to [7], which is a material for a cosmetic composition.
[9] A method of producing a cosmetic composition, including using the aqueous dispersion composition described in any one of [1] to [7]."

### [Effects of the Invention]

According to the present invention, it is possible to provide an aqueous dispersion composition in which hydrophobic powders are stably and uniformly dispersed in an aqueous phase, and which has excellent handling workability, and can exhibit sufficient water repellency, water resistance, sebum resistance, and the like when compounded in a cosmetic composition or the like. Furthermore, it is possible to provide a method of producing a cosmetic composition including using a material for a cosmetic composition containing the aqueous dispersion composition and the aqueous dispersion composition.

### [BRIEF DESCRIPTION OF THE DRAWINGS]

FIG. 1 is a diagram illustrating light transmittance in an ultraviolet region of an aqueous dispersion composition of Example 1, Example 2, and Comparative Example 1.
FIG. 2 is a diagram illustrating light transmittance in a visible light region of an aqueous dispersion composition of Example 1, Example 2, and Comparative Example 1.

### [Preferred Mode for Carrying Out the Invention]

Hereinafter, an aqueous dispersion composition of the present invention will be described below in detail. In the present invention, the aqueous dispersion composition is a composition in which a hydrophobic powder is dispersed in an aqueous medium and generally has fluidity, and therefore may also be referred to as a "water-based slurry". Furthermore, the aqueous medium of the present invention refers to an aqueous dispersion medium containing water, one or more alcohols selected from polyhydric alcohols and ethyl alcohols that are uniformly miscible with water, and salts such as basic substances, and the like, and is particularly preferably substantially free of an oil agent/oil-based material for a cosmetic composition capable of forming an independent oil phase without being miscible with water such as an oil agent. Note that, "substantially free" is preferably an amount such that the component is not intentionally added in the composition, and is particularly preferably in the range of less than 5% by mass, less than 1% by mass, or 0.1% by mass or less of the total composition to less than the detection limit. Further, the aqueous dispersion composition in the present invention contains components (A) to (E) described later in an amount of 80% by mass or more, suitably 90% by mass or more of the total composition, and since the composition is substantially free of an oil agent capable of forming an oil phase, while it is useful as a material for a cosmetic composition, it is distinguishable from the cosmetic composition containing oil-in-water emulsions and the like in terms of the composition thereof.

### [Aqueous dispersion composition]

The aqueous dispersion composition of the present invention may contain at least (A) carboxylic acid-modified silicone in a liquid form at 50°C, (B) a hydrophobic powder, (C) a basic substance, and (D) water; and optionally, (E) one or more alcohols selected from polyhydric alcohols and ethyl alcohols, wherein 80% by mass or more of a total composition is formed of the components (A) to (E), and the component (B) is contained in a range of 10% to 70% by mass of the total composition, and the component (A) is contained in a range of 5 to 30 parts by mass with respect to 100 parts by mass of the component (B). Suitably, 90% by mass or more of a total composition is formed of the components (A) to (E), and the content of water that is the component (D) is in a range of 10% to 80% by mass with respect to the total composition. In addition, as described above, the aqueous dispersion composition of the present invention is a composition in which a hydrophobic powder is dispersed in an aqueous medium, and is preferably substantially free of an oil agent capable of forming an oil phase. Hereinafter, each component and the compounding amount thereof will be described.

### [(A) Carboxylic acid-modified silicone]

The component (A) is at least one type of carboxylic acid-modified silicone in a liquid form at 50°C, and may be in a liquid form at 50°C and one atmosphere, and for example, it may be a solid at room temperature (25°C).

In the present invention, the carboxylic acid-modified silicone is a component that functions as a dispersant of (B) the hydrophobic powder, and when being used in a certain quantitative range with respect to hydrophobic powder, and used in combination with (C) the basic substance, it is possible to form an aqueous dispersion composition in which the hydrophobic powder is uniformly and stably dispersed in the aqueous phase.

The carboxylic acid-modified silicone contained in the cosmetic composition of the present invention is not particularly limited as long as it is an organosiloxane in which at least one carboxyl group-containing organic group is introduced to a side chain or an end, as long as the carboxylic acid-modified silicone is in a liquid form at 50°C. Preferably, the carboxyl group-containing organic group is introduced into the side chain of the organosiloxane.

Therefore, examples of the carboxylic acid-modified silicone include those in which a silicone main chain is grafted with a carboxyl group-containing organic group; a carboxyl group-containing organic group is added to one end of the silicone main chain; a carboxyl acid group-containing organic group is added to both ends of the silicone main chain; a carboxyl acid group-containing organic group is added to both ends of the silicone main chain, and the carboxyl group-containing organic group is further grafted; a silicone main chain grafted with a silicone chain (including a siloxane macromonomer bonded by a silalkylene bond) and a carboxyl group-containing organic group; and a silicone main chain or the end has a siloxane modifying group having a carbosiloxane dendrimer structure and a carboxyl group-containing organic group, and optionally, carboxylic acid-modified silicone having a long-chain alkyl group having 6 or more carbon atoms can be exemplified. The carboxylic acid-modified silicone in which a silicone main chain is grafted with a carboxyl group-containing organic group is most suitable.

A linking group may be present between a carboxyl group and a silicon atom, and examples of the linking group include divalent or higher valent organic groups such as an alkylene group which may have a hetero atom and a polyoxyalkylene group, but are not particularly limited. Furthermore, the (n-1) carboxyl group may be carboxylic acid-modified silicone bonded to a silicon atom by an n-valent linking group (n is a number of equal to or larger than 3). Specifically, the silicone having a carboxyl group on the main chain or side chain of the silicone via the following linking group is included in the carboxylic acid-modified silicone of the present invention.

Organopolysiloxane having a silicon-bonded carboxyl group-containing organic group disclosed in Japanese Translation of PCT International Application Publication No. H11-504665: 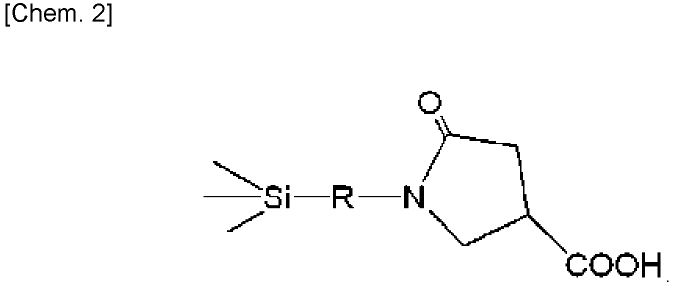 (wherein, R represents a C₁-C₁₂ alkylene group, a C₁-C₁₂ alkyleneoxy group, an oxygen atom, a sulfur atom,-NH-, or -NR'- (R' is a C₁-C₆ alkyl group), or a divalent group containing a combination of these),
Organopolysiloxane having any of the following carboxyl group-containing organic groups, disclosed in Japanese Unexamined Patent Application Publication No. 2002-114849: 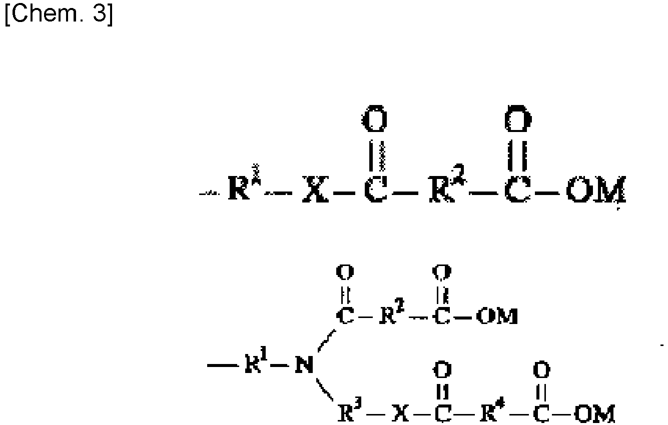 (wherein, R¹ to R²⁴ represent linear or branched chain alkylene groups, alkenylene groups, or arylene groups having 2 to 22 carbon atoms, which are the same or different and may have a substituent containing a hetero atom, X represents-O-or NH-, and M represents a hydrogen atom),
Organopolysiloxane having a carboxyl group-containing organic group disclosed in Japanese Translation of PCT International Application Publication No. 2005-524747: 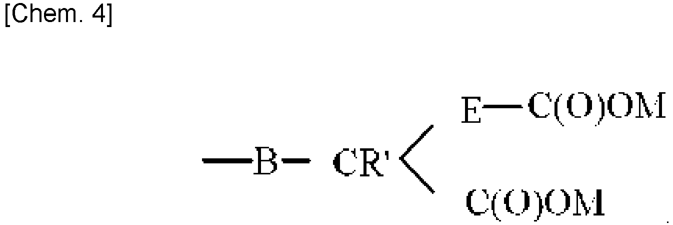 (wherein B represents an alkylene residue substituted with one or more alkyl groups having 2 to 30 carbon atoms and optionally 1 to 30 carbon atoms,
R' represents a hydrogen atom or an alkyl group having 1 to 30 carbon atoms, E is an alkylene residue that is absent or is substituted with one or more alkyl groups having 1 to 5 carbon atoms, preferably 1 to 3 carbon atoms, and optionally 1 to 30 carbon atoms; and M is a hydrogen atom), Organopolysiloxane having the following carboxyl group-containing organic group represented by the following average composition formula, disclosed in Japanese Unexamined Patent Application Publication No. 2009-263643:
[Chem. 5]

R¹ₐR²_{b}R³_{c}SiO_{(4-a-b-c)/2} (1)

[wherein, R¹ is a group selected from an alkyl group having 1 to 30 carbon atoms, a phloroalkyl group having 1 to 30 carbon atoms, an aryl group having 6 to 30 carbon atoms, and an aralkyl group having 6 to 30 carbon atoms,
R² is a group represented by Formula (2) below, and when c is 0, R² is bonded to at least one end of the organopolysiloxane, 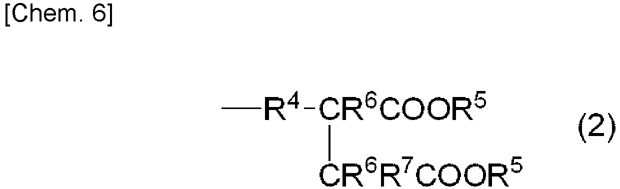 (wherein R⁴ is a divalent hydrocarbon group having or not having an oxygen atom having 2 to 20 carbon atoms, R⁵ is a hydrogen atom, R⁶s each independently represent a hydrogen atom or an alkyl group having 1 to 6 carbon atoms, and R⁷ is a hydrogen atom or an alkyl group having 1 to 6 carbon atoms),
R³ is a group represented by Formula (3) below: 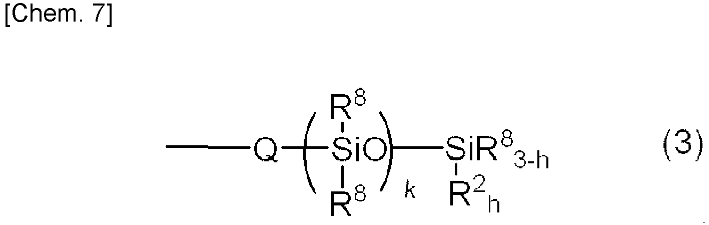 (wherein, R² is as described above, R⁸s each independently represent the group selected from an alkyl group having 1 to 30 carbon atoms, a fluoroalkyl group having 1 to 30 carbon atoms, an aryl group having 6 to 30 carbon atoms, and an aralkyl group having 6 to 30 carbon atoms, Q is C_{d}H_{2d} (here, d is an integer of 1 to 5, preferably an integer of 2 to 4) or an oxygen atom, k is an integer of 0 to 500, preferably 1 to 100, and more preferably 5 to 60, and h is an integer of 0 to 3 and preferably 0).

Particularly suitable examples of the carboxylic acid-modified silicone used in the present invention include carboxylic acid-modified silicone in which at least one silicon atom on the side chain or the end of the silicone main chain is bonded to a carboxyl group-containing organic group represented by a general formula: -R¹-(OR²)p-(O)w-R³-COOH, (wherein, R¹ represents a linear or branched alkylene group having 2 to 22 carbon atoms, R² represents a linear or branched alkylene group having 2 to 4 carbon atoms, R³ represents a bond (-) or a linear or branched alkylene group having 1 to 22 carbon atoms, p represents a number from 0 to 200, and w represents a number of 0 or 1).

In the general formula representing the carboxyl group-containing organic group, R¹ is a linear or branched alkylene group having 2 to 22 carbon atoms, preferably a linear alkylene group having 2 to 12 carbon atoms, and particularly preferably a linear alkylene group having 2 to 10 carbon atoms. Examples thereof include ethylene, propylene, trimethylene, butylene, pentamethylene, hexamethylene, heptamethylene, octamethylene, nonamethylene, decamethylene, undecamethylene, dodecamethylene, tridecamethylene, tetradecamethylene, pentadecamethylene, and hexadecamethylene groups.

In addition, examples of the linear or branched alkylene group having 2 to 4 carbon atoms of R² include ethylene, propylene, trimethylene and butylene groups, and an ethylene group is particularly preferable.

Examples of the linear or branched alkylene group having 1 to 22 carbon atoms of R³ include ethylene, ethylethylene, propylene, trimethylene, tetramethylene, pentamethylene, hexamethylene, heptamethylene, octamethylene, nonamethylene, decamethylene, undecamethylene, dodecamethylene, tridecamethylene, tetradecamethylene, pentadecamethylene, and hexadecamethylene groups. Among these, those having 1 to 12 carbon atoms, particularly those in which the sum of the carbon atoms of R¹ and R³ is 2 to 22 are preferable.

p represents the number of 0 to 200, and the number of 0 to 20 is preferable, and the number of 0 to 10 is particularly preferable. In addition, w represents the number of 0 or 1, and is preferably 0. Note that, when p and w are both 0, the carboxyl group-containing organic group is represented by the structural formula -(CₙH₂ₙ)-COOH, and the carboxyl group-containing organic group preferably has a structure in which one carboxyl group is bonded to a silicon atom via a linear or branched alkylene group having 3 to 44 carbon atoms. In the formula, n is a number of 3 to 44, preferably a number of 3 to 20, and particularly preferably a number of 3 to 16.

Examples of the carboxylic acid-modified silicone used in the present invention include organopolysiloxane represented by the following structural formula (1): 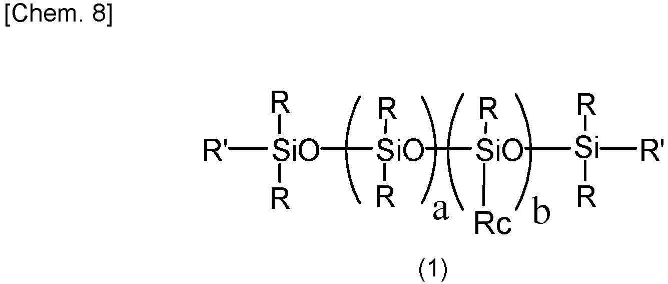 (wherein Rc represents a carboxyl group-containing organic group represented by a general formula: -R¹-(OR²)p-(O)w-R³-COOH, R represents the same or different alkyl or alkoxy group, having 1 to 22 carbon atoms, or phenyl group, R' is Rc or R, and each of a and b is a number in the range of 0 or more, and a + b is a number in the range of 0 to 1000. here, when b = 0, at least one of R' is Rc). In particular, the carboxylic acid-modified silicone disclosed in Japanese Unexamined Patent Application Publication No. 8-109263 and some of (other than those having a siloxane dendron structure) of the carboxylic acid-modified silicone disclosed in PCT International Publication No. WO 2009/22621 are represented by the structural formula (1) and are included in the carboxylic acid-modified silicone suitably used in the present invention.

Suitable examples of the carboxylic acid-modified silicone represented by structural formula (1) include carboxylic acid-modified silicone in which a + b is a number in the range 0 to 500, particularly, b > 0, and a carboxyl group-containing organic group represented by the general formula: -R¹-(OR²)p-(O)w-R³-COOH is grafted with the silicone main chain, and carboxylic acid-modified silicone in which b = 0, and R' at both ends of the silicone main chain is a carboxyl group-containing organic group represented by the general formula: -R¹-(OR²)p-(O)w-R³-COOH. In the present invention, a particularly suitable carboxylic acid-modified silicone is carboxylic acid-modified silicone having a large number of carboxyl group-containing organic groups in the side chain moiety, and it is preferable that b > a, and it is more suitable that b > 0 and a = 0. "b > a" means that more than half of the side chain moieties have siloxane units having a carboxyl group-containing organic group, and a + b is preferably a number in the range of 1 to 500. Furthermore, when a = 0, if b > 0, all of the siloxane units in the side chain moiety having a carboxyl group-containing organic group, and b is most preferably a number in a range of 1 to 200 or a number in a range of 1 to 50.

In structural formula (1), R is preferably a methyl group, an alkoxy group, or a phenyl group, however, from the viewpoint of compounding stability with an organic oil agent such as a hydrocarbon oil or an organic material for a cosmetic composition (in particular, a UV absorber), R may have a long chain alkyl group with 6 to 22 carbon atoms in a part. A degree of modification by the carboxyl group-containing organic group is not particularly limited, and if a + b is a number in the range of 0 to 500, it is preferable to have an average of 2 to 100 of the carboxyl group-containing organic groups in the molecule, including a case where the carboxyl group-containing organic group is bonded to both ends of the silicone main chain.

In the present invention, such carboxylic acid-modified silicone can be produced by known methods such as a method of subjecting dimethylpolysiloxane having a Si-H group and an unsaturated carboxylic acid ester compound to addition reaction under a platinum catalyst and to saponification to form carboxylic acid; a method of subjecting dimethylpolysiloxane having a Si-H group to addition reaction of unsaturated carboxylic acid silyl ester or allyloxycarboxylic acid silyl ester under a platinum catalyst, and to obtain the desired product by hydrolysis after the reaction; and a method of obtaining carboxylic acid-modified silicone at both ends by an equilibrium reaction using bis(hydroxycarbonylethyl) tetramethyldisiloxane with cyclic siloxane and an acidic catalyst (Silicone Handbook, edited by Kunio Ito, NIKKAN KOGYO SHIMBUN,LTD. pp. 166-167).

Further, in the present invention, as the carboxylic acid-modified silicone represented by the structural formula (1), commercially available carboxylic acid-modified silicone can be used as it is or after removing the solvent. Specific examples thereof include BY16-880, BY16-750, and FZ-3516 (available from Dow Toray Co., Ltd.), TSF 4770, and TSF 4771 (available from Momentive Performance Materials), X-22-162A, X-22-162C, X-22-3701E, and X-22-3710 (available from Shin-Etsu Chemical Co., Ltd.), and the like.

From the viewpoint of technical effects of the present invention, the (A) carboxylic acid-modified silicone is preferably in a liquid form at room temperature (25°C). Note that, the (A) carboxylic acid-modified silicone may be in a liquid form at room temperature (25°C) and one atmosphere. The carboxylic acid-modified silicone that is in a liquid form at room temperature (25°C) has advantages in that dispersion effects are excellent on the (B) hydrophobic powder and no heating operation is required when forming the aqueous dispersion composition.

It is particularly preferable that, in the structural formula (1), the (A) carboxylic acid-modified silicone is carboxylic acid-modified silicone which is in a liquid form at room temperature (25°C) in which a + b is a number in a range of 2 to 20, and a/b is in a range of 0.3 to 3.0. By using the carboxylic acid-modified silicone having such a structure, the uniform dispersibility of the (B) hydrophobic powder in the aqueous phase is further improved. With this, when the aqueous dispersion composition containing the carboxylic acid-modified silicone is used as a material for a cosmetic composition, there is an advantage in that sufficient functions such as water repellency, water resistance, and sebum resistance can be effectively realized. There is also an advantage in that the uniform dispersion state of the (B) hydrophobic powder in the aqueous phase is maintained for a long period of time.

It is most suitable that the (A) carboxylic acid-modified silicone is represented by the following structural formula (2): 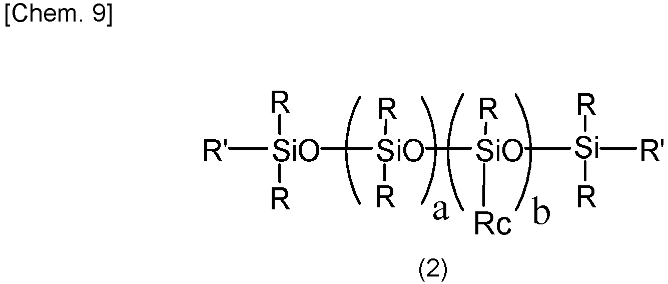 (wherein:
Rc represents a carboxyl group-containing organic group represented by a general formula: -R¹-(OR²)p-(O)w-R³-COOH, (R¹ represents a linear or branched alkylene group having 2 to 22 carbon atoms, R² represents a linear or branched alkylene group having 2 to 4 carbon atoms, R³ represents a bond (-) or a linear or branched alkylene group having 1 to 22 carbon atoms, p represents a number from 0 to 200, and w represents a number of 0 or 1),
R represents the same or different alkyl or alkoxy group, having 1 to 22 carbon atoms, or phenyl group,
R' is Rc or R,
each of a and b is a positive number, a ≥ 2 is preferable, and b ≥ 2 is preferable, a + b is a number in the range of 2 to 20, preferably 2 to 15, and more preferably 2 to 10, and
a/b is in the range of 0.3 to 3.0, preferably 0.3 to 2.5, more preferably 0.3 to 2.0, and even more preferably 0.5 to 2.0).

In the general formula representing the carboxyl group-containing organic group in the structural formula (2), R¹ is a linear alkylene group having 2 to 22 carbon atoms, preferably a linear alkylene group having 2 to 12 carbon atoms, and particularly preferably a linear alkylene group having 2 to 10 carbon atoms. Examples thereof include ethylene, propylene, trimethylene, butylene, pentamethylene, hexamethylene, heptamethylene, octamethylene, nonamethylene, decamethylene, undecamethylene, dodecamethylene, tridecamethylene, tetradecamethylene, pentadecamethylene, and hexadecamethylene groups.

In addition, examples of the linear or branched alkylene group having 2 to 4 carbon atoms of R² include ethylene, propylene, trimethylene and butylene groups, and an ethylene group is particularly preferable.

Examples of the linear or branched alkylene group having 1 to 22 carbon atoms of R³ include ethylene, ethylethylene, propylene, trimethylene, tetramethylene, pentamethylene, hexamethylene, heptamethylene, octamethylene, nonamethylene, decamethylene, undecamethylene, dodecamethylene, tridecamethylene, tetradecamethylene, pentadecamethylene, and hexadecamethylene groups. Among these, those having 1 to 12 carbon atoms, particularly those in which the sum of the carbon atoms of R¹ and R³ is 2 to 22 are preferable.

p represents the number of 0 to 200, and the number of 0 to 20 is preferable, and the number of 0 to 10 is particularly preferable. In addition, w represents the number of 0 or 1, and is preferably 0. Note that, when p and w are both 0, the carboxyl group-containing organic group is represented by the structural formula -(CₙH₂ₙ)-COOH, and the carboxyl group-containing organic group preferably has a structure in which one carboxyl group is bonded to a silicon atom via a linear or branched alkylene group having 3 to 44 carbon atoms. In the formula, n is a number of 3 to 44, preferably a number of 3 to 20, and particularly preferably a number of 3 to 16.

The (A) carboxylic acid-modified silicone represented by structural formula (2) is not particularly limited as long as the at least one carboxyl group-containing organic group is a side chain or the end introduced organosiloxane. Preferably, the carboxyl group-containing organic group is introduced into the side chain of the organosiloxane.

Therefore, examples of the (A) carboxylic acid-modified silicone represented by the structural formula (2) include those in which a silicone main chain is grafted with a carboxyl group-containing organic group as a side chain; a carboxyl group-containing organic group is added to one end of the silicone main chain; a carboxyl acid group-containing organic group is added to both ends of the silicone main chain; and a carboxyl acid group-containing organic group is added to both ends of the silicone main chain, and the carboxyl group-containing organic group is further grafted as a side chain, and optionally, carboxylic acid-modified silicone having a long-chain alkyl group having 6 or more carbon atoms can be exemplified. The carboxylic acid-modified silicone in which a silicone main chain is grafted with a carboxyl group-containing organic group is most suitable as a side chain.

The (A) carboxylic acid-modified silicone represented by the structural formula (2) is preferably carboxylic acid-modified silicone in which R' is R and a carboxyl group-containing organic group represented by the general formula: -R¹-(OR²)p-(O)w-R³-COOH is grafted with the silicone side chain; more preferably carboxylic acid-modified silicone in which R' is R and the silicone side chain has a plurality of the aforementioned carboxyl group-containing organic groups; and still more preferably carboxylic acid-modified silicone in which R' is R, the silicone side chain has a plurality of the aforementioned carboxyl group-containing organic groups, and a/b = 1.

In the structural formula (2), R is preferably a methyl group, an alkoxy group, or a phenyl group.

In the present invention, such carboxylic acid-modified silicone can be produced by known methods such as a method of subjecting dimethylpolysiloxane having a Si-H group and an unsaturated carboxylic acid ester compound to addition reaction under a platinum catalyst and to saponification to form carboxylic acid; a method of subjecting dimethylpolysiloxane having a Si-H group to addition reaction of unsaturated carboxylic acid silyl ester or allyloxycarboxylic acid silyl ester under a platinum catalyst, to obtain the desired product by hydrolysis after the reaction; and a method of obtaining carboxylic acid-modified silicone at both ends by an equilibrium reaction using bis(hydroxycarbonylethyl) tetramethyldisiloxane with cyclic siloxane and an acidic catalyst (Silicone Handbook, edited by Kunio Ito, NIKKAN KOGYO SHIMBUN,LTD. pp. 166-167). Furthermore, particularly, examples of the suitable carboxylic acid-modified silicone of the present invention include those available from the trade name ES-5800 Formulation Aid (available from Dow Toray Co., Ltd.), and the like.

In the aqueous dispersion composition of the present invention, the content of the (A) carboxylic acid-modified silicone is required to be in the range of 5 to 30 parts by mass with respect to 100 parts by mass of the component (B), and a range of 10 to 25 parts by mass is more preferable. If the compounding amount of the component (A) is less than the lower limit described above, dispersion of the hydrophobic powder in the aqueous phase may be insufficient, and if the compounding amount of the component (A) exceeds the upper limit described above, it may not be possible to compound the component (B) in the aqueous dispersion composition in a sufficient amount.

### [(B) Hydrophobic powder]

The component (B) of the present invention is a hydrophobized powder. Since a surface of such hydrophobic powder is hydrophobized, it is difficult to disperse in the aqueous phase; however, in the aqueous dispersion composition of the present invention, it is possible to realize a composition in which a large amount of hydrophobic powder is uniformly and stably dispersed in the aqueous phase in the range of 10% to 70% by mass, suitably in the range of 15% to 65% by mass, more suitably in the range of 15% to 60% by mass of the total composition.

Examples of the powder include inorganic powders, organic powders, surfactant metal salt powders (metal soaps), colored pigments, pearl pigments, and metal powder pigments; and composites of these can also be used. Specifically, inorganic powders are not particularly limited, and examples thereof include commonly used zinc oxide, titanium oxide, zirconium oxide, cerium oxide, magnesium oxide, barium sulfate, calcium sulfate, magnesium sulfate, calcium sulfate, magnesium carbonate, talc, mica, kaolin, sericite, muscovite, synthetic mica, phlogopite, lepidolite, biotite, lithia mica, silicic acid, silicic anhydride, aluminum silicate, sodium silicate, sodium magnesium silicate, magnesium silicate, magnesium aluminum silicate, calcium silicate, barium silicate, strontium silicate, metal tungstate, hydroxyapatite, vermiculite, hydrargilite, bentonite, montmorillonite, hectorite, zeolite, ceramic powder, dicalcium phosphate, alumina, aluminum hydroxide, boron nitride, boron nitride, and the like; organic powders include polyamide powder, polyester powder, polyethylene powder, and polypropylene powder, polystyrene powder, polyurethane powder, polystyrene powder, benzoganamine powder, polymethylbenzoganamine powder, polytetrafluoroethylene powder, polymethyl methacrylate powder, cellulose, silk powder, nylon powder, 12-nylon, 6-nylon, silicone powder, polymethylsilsesquioxane spherical powder, styrene/acrylic acid copolymer, divinylbenzene/styrene copolymer, vinyl resin, urea resins, phenol resin, fluororesin, silicon resin, acrylic resin, melamine resin, epoxy resin, polycarbonate resin, microcrystalline fiber powder, starch powder, lauroyl lysine, and the like; surfactant metal salt powders include zinc stearate, aluminum stearate, calcium stearate, magnesium stearate, zinc myristate, magnesium myristate, zinc palmitate, zinc laurate, zinc cetyl phosphate, calcium cetyl phosphate, sodium zinc cetyl phosphate, and the like; colored pigments include inorganic red pigments such as red oxide, iron oxide, iron hydroxide, iron titanate, inorganic brown pigments such as γ-iron oxide, inorganic yellow pigments such as yellow iron oxide, black iron oxide, inorganic black pigments such as carbon black, inorganic purple pigments such as manganese violet, cobalt violet, and the like, inorganic green pigments such as chromium hydroxide, chromium oxide, cobalt oxide, cobalt titanate, and the like, inorganic blue pigments such as prussian blue, ultramarine blue, and the like; those obtained by laking tar dyes such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, Orange No. 207, and the like, or those obtained by laking natural dyes such as carmine acid, lacquemic acid, carthamine, braziline, chrosine and the like; pearl pigments such as titanium oxide-coated mica, titanated mica, iron oxide-treated titanated mica, titanium oxide-coated mica, bismuth oxychloride, titanium oxide-coated bismuth oxychloride, titanium oxide-coated talc, fish scale foil, titanium oxide-coated colored mica and the like; oxidized titanium oxide coated mica, oxychlorinated bismuth, titanium oxide coated bismuth oxychloride, titanium oxide coated tantalum foil, fish scaly foil, titanium oxide coated colored mica, and the like; metal powder pigments such as aluminum, gold, silver, copper, platinum, stainless steel, and the like.

A silicone elastomer powder may also be used as the hydrophobic powder. The silicone elastomer powder is a crosslinked product of a liner diorganopolysiloxane mainly composed of a diorganosiloxy unit (D unit), and can be suitably obtained by crosslinking an organohydrogenpolysiloxane having a silicon-bonded hydrogen atom at the side chain or end and a diorganopolysiloxane having an unsaturated hydrocarbon group such as an alkenyl group at the side chain or end under a hydrosilylation reaction catalyst. Since the silicone elastomer powder is soft, elastic, and excellent in oil absorption compared to the silicone resin powder composed of a T unit and a Q unit, the silicone elastomer powder can absorb oil and fat on the skin and prevent cosmetic collapse.

The silicone elastomer powder may have various shapes, such as spherical, flat, or amorphous. The silicone elastomer powder may be in the form of an oil dispersion. The cosmetic composition of the present invention is a silicone elastomer powder having a particle shape, a primary particle diameter thereof determined by observation using an electron microscope and/or an average primary particle diameter measured by laser diffraction/scattering method falls within a range of 0.1 to 50 µm, and a silicone elastomer powder having a spherical primary particle shape can be suitably compounded. The silicone elastomer constituting the silicone elastomer powder is preferably a silicone elastomer having a hardness of 80 or less, more preferably 65 or less according to JIS K 6253 "Hardness testing method for rubber, vulcanized or thermoplastic" as measured by type-A durometer. In addition, these silicone elastomer powders may optionally be subjected to a surface treatment with a silicone resin, silica, or the like.

The powder used in the present invention is particularly preferably an inorganic powder having ultraviolet absorption/scattering capability on titanium oxide, zinc oxide, iron oxide, cerium oxide, and composites thereof. The composite refers to a surface treatment powder, a powder in which other inorganic particles are dispersed within an inorganic powder, and the like.

The inorganic powder having an ultraviolet light shielding effect is widely used in applications for cosmetic composition and, therefore, is preferable from the viewpoint of ease of compounding into a water-based cosmetic composition. Furthermore, the use of these inorganic powders is preferable in that an inorganic thin film having an ultraviolet shielding effect can be easily formed. Among these, one type or two or more types of hydrophobic powders selected from hydrophobic fine particulate titanium oxide and hydrophobic fine particulate zinc oxide are particularly preferable. Here, the particle size of the hydrophobic fine particulate titanium oxide and hydrophobic fine particulate zinc oxide is preferably 1 to 200 nm in terms of ultraviolet protection effect and dispersibility, and more preferably 10 to 80 nm.

The inorganic powder used in the present invention may be a composite powder in which the surface is coated with another inorganic material. The surface treatment material in this case may be a known inorganic surface treatment material, and examples thereof include zinc oxide, titanium oxide, cerium oxide, iron oxide, barium sulfate, hydrous silicic acid, silica, aluminum hydroxide, alumina, and the like. A coating amount thereof is preferably from 1% by weight to 25% by weight with respect to the total inorganic powder.

When the inorganic powder is an ultraviolet light shielding particle, the average particle size is preferably 10 to 200 nm. An ultraviolet light blocking particle having such a particle size is particularly suitable in that the visible light transparency is high and an ultraviolet radiation-shielding area is suitable. In addition, when the particle size exceeds 200 nm, there is a risk that the visible light transparency will be poor, and the ultraviolet light shielding capability may also deteriorate. Furthermore, when the particle size is less than 10 nm, it is not preferable in that the ultraviolet shielding capability may deteriorate. In addition, when the inorganic powder is not an ultraviolet light blocking particle or ultraviolet light blocking property is not necessary, the particle size may be a size optimum for using the particles.

The shape of the inorganic powder is not particularly limited, and any shape such as a spherical shape, a rod shape, a needle shape, a spindle shape, a plate shape, or the like can be used. Note that in the case of rod-shaped, needle-shaped, and spindle-shaped particles, the average particle size is defined by the length on the short axis side, and in the case of plate-shaped particles, the average particle size is defined by the average of the diagonal length of the surface.

The hydrophobizing treatment of the inorganic powder is not particularly limited, but means that the powder is treated with various hydrophobized surface treatment agents. Examples of the hydrophobizing treatment include a methyl hydrogen polysiloxane (methylcon in Japanese cosmetics label name) treatment, a (dimethicone/methicone) copolymer (hydrogen dimethicone in Japanese cosmetics label) treatment, a dimethylpolysiloxane (dimethicone in Japanese cosmetics label) treatment, a carboxylic acid-modified silicone treatment, a silicone resin treatment, a silicone gum treatment, an acrylic silicone treatment, an organosiloxane treatment such as a fluorinated silicone treatment; a metal soap treatment such as a zinc stearate treatment, a silane coupling agent treatment; a silane treatment such as an alkylsilane treatment, a fluorine compound treatment such as a perfluoroalkyl phosphate ester salt; a perfluoro-ether treatment; an amino acid treatment such as an N-lauroyl-L-lysine treatment; an oil agent treatment such as a squalane treatment; an acrylic acid treatment such as an alkyl acrylate treatment; and a titanate-based treatment such as alkyl titanate, and the like, and two or more of these treatments can be also used in combination.

Among these treatments, the silane treatment, the treatment with a silicone compound, or the titanate treatment is preferable from the viewpoint of water resistance of the obtained aqueous dispersion composition and ease of dispersion with the carboxylic acid-modified silicone. Among them, it is particularly preferable to perform a treatment with a titanium coupling agent such as methylhydrogenpolysiloxane, a (dimethicone/methicone) copolymer, dimethylpolysiloxane, alkylsilane, alkyl titanate, pyrophosphate type titanate, phosphorous acid type titanate, and amino acid type titanate. When these reactive hydrophobizing agents are used, hydrophobizing agents are less likely to liberation and consequently aggregation of the inorganic powders is effectively suppressed because the hydrophobizing agents form a chemical bond on the surface of the obtained hydrophobic powder.

The degree of the hydrophobizing treatment is optional, but it is suitable that the treatment with the aforementioned hydrophobizing agent is performed at a ratio of 2% to 12% by mass, and suitably 3% to 10% by mass, with respect to the total amount of the treated inorganic powder.

### [(C) Basic substance]

The aqueous dispersion composition of the present invention contains at least one (C) basic substance. The component (C) is capable of anionizing the carboxylic acid modified moiety of the aforementioned (A) carboxylic acid-modified silicone, and improving the function of the (A) carboxylic acid-modified silicone as a surfactant/powder dispersant. In particular, the aqueous dispersion composition of the present invention contains (B) hydrophobic powder, however, by using the component (A) and the component (C) in combination, it is possible to favorably disperse the component (B) in the aqueous phase as compared to using the component (A) alone so that it contributes to not only improving the uniform dispersibility and stability of the aqueous dispersion composition body, but also achieving sufficient water repellency, water resistance, and sebum resistance of the cosmetic composition compounded with the aqueous dispersion composition as a material for a cosmetic composition.

The basic substance used in the present invention is not particularly limited as long as the basic substance is a compound that exhibits basicity when dissolved in water, and various types of inorganic compounds and organic compounds can be used. One or more types of the basic substances may be compounded.

Examples of the organic compounds include monoethanolamine, triethanolamine, 2-amino-2-methyl-1,3-propanediol, aminomethylpropanol, aminomethyl propanol, 2-amino-2-hydroxymethyl-1,3-propanediol, arginine, guanidine, and the like.

Examples of the inorganic compounds include sodium hydroxide, sodium carbonate, potassium hydroxide, potassium carbonate, calcium hydroxide, calcium carbonate, ammonia, and the like. Among these, potassium hydroxide can be particularly suitably used.

The compounding amount of the basic compound in the aqueous dispersion composition of the present invention is not particularly limited, and when the base is a monovalent base per 1 mol of the carboxylic acid groups contained in the compounded carboxylic acid-modified silicone, the carboxylic acid group/monovalent base (molar ratio) is preferably 1/0.5 to 1/1.5.

The pH of the aqueous dispersion composition of the present invention may be acidic or alkaline, and from the viewpoint of anionizing the carboxylic acid-modified site of the aforementioned (A) carboxylic acid-modified silicone and improving the dispersibility on the hydrophobic powder as the component (B), is preferably weakly acidic to alkaline, and specifically, the pH of the total composition is preferably in the range of 6.5 to 14.0, may be in the range of pH 7.1 to 10.0, and is more preferably in the range of ph 7.2 to 9.5.

### [(D) water]

Water is a main dispersion medium of the aqueous dispersion composition of the present invention, and is miscible with (E) polyhydric alcohol, which is also an aqueous dispersion medium, to form an aqueous phase and form a structure in which the hydrophobic powder as the component (B) is stably dispersed in the aqueous phase under the presence of the component (A) and the component (C).

The compounding amount of the water in the aqueous dispersion composition of the present invention is not particularly limited, and is preferably in the range of 10% to 80% by mass, more preferably in the range of 15% to 70% by mass, and still more preferably in the range of 20% to 65% by mass, based on the total mass of the composition. In particular, when the content of water is less than the lower limit described above, the total composition becomes more likely to be thickened, and in particular, when the compounding amount of the (E) polyhydric alcohol is large, the composition is flammable and handling workability as a material for a cosmetic composition may deteriorate. On the other hand, when the compounding amount of water exceeds the upper limit described above, in some cases, the hydrophobic powder as the component (B) cannot be compounded in a sufficient amount in the composition.

### [(E) One or more alcohols selected from polyhydric alcohols and ethyl alcohols]

The (E) one or more alcohols selected from polyhydric alcohols and ethyl alcohols have any configuration in the aqueous dispersion composition according to the present invention. The aqueous dispersion composition of the present invention can be formed even if the component (E) is absent; however, from the viewpoint of dispersion stability, at least (E) one or more alcohols selected from polyhydric alcohols and ethyl alcohols can be contained and is preferable. The polyhydric alcohol and/or ethyl alcohol is miscible with the water described above to form an aqueous phase and form a structure in which the hydrophobic powder as the component (B) is stably dispersed in the aqueous phase under the presence of the component (A) and the component (C).

In particular, by containing one or more alcohols selected from polyhydric alcohols and ethyl alcohols, when the aqueous dispersion composition of the present invention is used as a material for a cosmetic composition, the moisturizing feeling and the feeling of use can be adjusted, and in preparing the aqueous dispersion composition of the present invention, the component (A) and hydrophobic powder are premixed with polyhydric alcohol and/or ethyl alcohol and then mixed with other components to prepare the aqueous dispersion composition, and thereby the hydrophobic powder can be well dispersed in the aqueous phase.

Examples of the polyhydric alcohols include sorbitol, xylitol, propylene glycol, dipropylene glycol, 1,3-butylene glycol, glycerin, diglycerin, polyethylene glycol, and the like, and these polyhydric alcohols can be used alone or in combination of two or more types. When the component (A) and the hydrophobic powder are pre-mixed together with the polyhydric alcohol, the liquid polyhydric alcohol can be uniformly dispersed in the aqueous phase. Among these, propylene glycol, dipropylene glycol, 1,3-butylene glycol, glycerin, diglycerin, and combinations thereof are preferable. Meanwhile, ethyl alcohol is sometimes referred to as ethanol, has the property of being uniformly dispersible in the aqueous phase, and can be used alone or in combination with the polyhydric alcohol. Specifically, the ethyl alcohol and the polyhydric alcohol can be mixed at any mass ratio of 100: 0 to 0: 100 or used alone in the present invention.

Since the polyhydric alcohol and/or the ethyl alcohol have any configuration in the aqueous dispersion composition according to the present invention, the compounding amount thereof may be 0% by mass. On the other hand, when a compounding amount of the polyhydric alcohol and/or ethyl alcohol is compounded in the aqueous dispersion composition of the present invention, the compounding amount thereof is not particularly limited, and is preferably 0.1% to 50% by mass, more preferably 0.5% to 45% by mass, still more preferably 1% to 40% by mass, and particularly preferably 2% to 35% by mass, based on the total mass of the aqueous dispersion composition.

### [Occupancy amount of components (A) to (E)]

The aqueous dispersion composition of the present invention contains the aforementioned components (A) to (E) of 80% by mass or more with respect to the total amount of the composition and contains the component (B) in a range of 10% to 60% by mass of the total composition. Furthermore, the aforementioned components (A) to (E) are more preferably 90% by mass or more in the total amount of the composition. Note that, the compounding amount of the component (E) may be 0% by mass.

The aqueous dispersion composition of the present invention can use the hydrophobic powder as a material for use in compositions such as cosmetic compositions, paints, inks, and the like. Therefore, if other components are compounded, an extra compound will be present when the dispersion of the present invention is used as a material, for example, the composition may not be used in a case where a substance which is not supposed to be added when compounded in the cosmetic composition, which is not preferable in that it lacks application.
It is also unfavorable in that it may cause unfavorable performance with other components used in combination in the formulation.

The aqueous dispersion composition of the present invention does not contain a component other than the aforementioned components in a proportion exceeding 20% by weight, and other components may be contained in a proportion of 20% by weight or less, more preferably 10% by weight or less, as long as the performance of the dispersion is not impaired. However, it is preferable not to add a compound or the like that may impair the stability of the dispersion. In particular, the addition of the oil agent is not preferable.

The component that may be added is not particularly limited, and examples thereof include preservatives. That is, the aqueous dispersion composition formed of the aforementioned components may cause spoilage depending on the storage conditions and the storage period. The preservatives may be added to prevent such spoilage. Such preservatives are not particularly limited, and examples thereof include glycol-based preservatives such as propylene glycol, butylene glycol, dipropylene glycol, pentylene glycol, hexylene glycol, and the like, parabenzoic acid esters such as methyl paraoxybenzoate and the like, piroctone olamine, phenoxyethanol, caprylsanpolyglyceryl-3, and the like. These can be added for each component within a range that exhibits antiseptic performance and does not adversely affect the performance of the dispersant.

The specific added amount of the preservative described above can be, for example, 20% by weight or less, and more preferably 10% by weight or less. Among the above preservatives, it is particularly preferable to use a polyhydric alcohol such as propylene glycol, butylene glycol, dipropylene glycol, pentylene glycol, and hexylene glycol. Two or more types of compounds may be used in combination from these groups. Among these, it is particularly preferable to use pentylene glycol and 1,3-butylene glycol. From the viewpoint of application described above, the content of the component other than the powder, water, dispersant, or preservative is preferably 1% or less. The above glycols are often used in cosmetics as moisturizers, but they also have antibacterial properties and therefore have an effect as preservatives. Furthermore, since these components are components that overlap with a part of the above component (E), by using polyhydric alcohol as the component (E), the storage stability and moisturizing function of the aqueous dispersion composition of the present invention can be improved without adding another preservative, moisturizer, or the like in some cases.

The aqueous dispersion composition of the present invention preferably does not contain components other than the aforementioned components and preservatives. This is because, by compounding other components, the stability can be adversely affected. It is also undesirable that components that are not supposed to be compounded when compounded in cosmetics may be contained.

The dispersion method of obtaining the dispersion of the present invention may be any known method capable of uniformly dispersing each of the components using mechanical force. A method using a bead mill, a method using a high pressure homogenizer, a method using three rolls, and the like are suitable.

The aqueous dispersion composition obtained in the present invention can be applied to make a thin film. For example, a zinc oxide film that is fixed with silica can be produced by applying a silicone-treated zinc oxide dispersion and heat treating with high heat, plasma, or the like. Further, when a dispersant such as a polyether or a polyether alkyl ether is used as a dispersant for a similar zinc oxide dispersion, a silicone-coated zinc oxide coating film can be easily produced by heating at a temperature lower than the decomposition temperature of the silicone coated with zinc oxide.

### [Use of aqueous dispersion composition: Material for cosmetic composition or the like]

In addition, the aqueous dispersion composition of the present invention can be compounded as is into the cosmetic compositions, paints, and inks. In this case, a cosmetic composition, an aqueous paint, and an ink composition can be obtained by mixing the various components used in the dispersion, cosmetic composition, paint, ink, and the like. In particular, the aqueous dispersion composition of the present invention is suitable as a material for a cosmetic composition that is compounded directly into a cosmetic composition.

The cosmetic composition obtained in this manner can suitably disperse the hydrophobic powder in the aqueous phase of the water-based composition or emulsion. As a result, a function such as sufficient water repellency, water resistance, sebum resistance, and the like can be obtained by a cosmetic composition or the like in which the water-based dispersion is compounded.

The cosmetic composition is not particularly limited, and by mixing the material for a cosmetic composition as necessary into the aqueous dispersion composition of the present invention, a base make-up cosmetic composition such as a sunscreen agent or the like; a base makeup cosmetic composition such as foundations; a point makeup cosmetic composition such as lipstick; and the like. That is, the aqueous dispersion composition of the present invention can be used directly in the production of cosmetic compositions.

The cosmetic composition can be in any form of an oil-based cosmetic composition, an aqueous cosmetic composition, an O/W cosmetic composition, or a W/O type cosmetic composition. Among these, a sunscreen agent can be particularly suitably used.

The cosmetic composition may be a combination of any aqueous component or oily component that can be used in the cosmetic field. The aqueous component and the oily component are not particularly limited, and examples thereof include oil agents, surfactants, moisturizers, higher alcohols, metal ion sequestering agents, natural and synthetic polymers, water-soluble and oil-soluble polymers, UV blocking agents, various extracts, coloring agents such as organic dyes, preservatives, antioxidants, pigments, thickeners, pH adjusters, fragrances, cooling agents, antiperspirants, bactericides, skin activators, and those containing components such as various powders.

These components are, for example, available without limitation as specifically disclosed in Patent Document 1 and Patent Document 2.

When the aqueous dispersion composition of the present invention is compounded into a paint, a resin in the paint may be curable or non-curable. The paint may be a solvent-based paint containing an organic solvent or a water-based paint in which a resin is dissolved or dispersed in water.

When the aqueous dispersion composition of the present invention is used as an added component to a paint composition, it can be used in combination with a coating film forming resin such as an acrylic resin, a polyester resin, and an epoxy resin; various pigments such as a coloring pigment, an extender pigment, and a bright pigment; and a curing catalyst, a surface conditioner, a defoamer, a pigment dispersant, a plasticizer, a film-forming aid, a UV absorber, and an antioxidant.

Also, the aqueous paint and aqueous ink composition thus obtained is preferable in that the hydrophobic powder is stably dispersed in the aqueous medium.

### [Example 1]

Hereinafter, the present invention will be described in more detail based on examples, but the present invention is not limited to these examples. The compounding amount of each component is "% by mass" ("% by weight") unless otherwise specified. The aqueous dispersion composition according to the examples or the like hereinafter is simply referred to as "aqueous dispersion".

The carboxylic acid-modified silicone (compound 1 and compound 2), which is component (A) of the present invention, was synthesized by the following method.

### [Synthesis Example 1]

In a flask equipped with a stirrer, a reflux condenser, a dropping funnel, and a thermometer, 230.67 g of trimethylsilyl undecylenate and 0.042 g of platinum-1,3-divinyl-1,1,3,3-tetramethyldisiloxane complex were added, and then 129.33 g of Si-H siloxane represented by the following structural formula was added dropwise while keeping a temperature range of 70°C to 80°C. 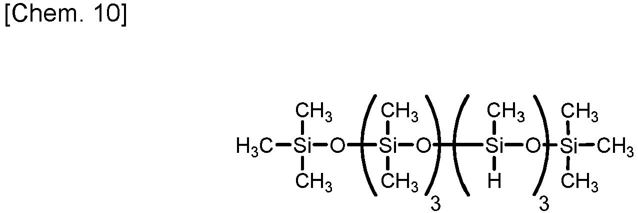 After dropping was complete, the mixture was aged for 2 hours at 110°C, and then the disappearance of the Si-H bond was confirmed by a hydrogen generation method. The low boiling point content was distilled off under reduced pressure. Thereafter, 90 g of deionized water was added, aged at reflux for 4 hours, and deprotection was performed. Thereafter, the low boiling point content was again removed under reduced pressure to obtain a compound 1. As a result of the analysis, it was confirmed that it was the compound 1 represented by the following chemical structural formula.

### Compound 1:

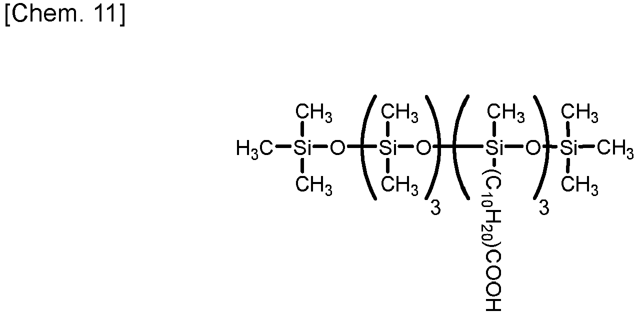

### (Synthesis Example 2)

In a flask equipped with a stirrer, a reflux condenser, a dropping funnel, and a thermometer, 100 g of 1,1,1,3,5,5,5-heptamethyltrisiloxane, and 0.02 g of toluene solution of platinum-1,3-divinyl-1,1,3,3-tetramethyldisiloxane complex were added, and 105 g of trimethylsilyl undecylenate was added dropwise while keeping a temperature range of 70°C to 100°C. After dropping was complete, the mixture was aged for 2 hours at 110°C, and then the disappearance of the Si-H bond was confirmed by a hydrogen generation method. The low boiling point content was distilled off under reduced pressure. Thereafter, water was added, aged at reflux for 4 hours, and deprotection was performed. Thereafter, the low boiling point content was again removed under reduced pressure to obtain a compound 2. As a result of the analysis, it was confirmed that the chemical structure of the compound 2 is shown in the following chemical formula:

### Compound 2:

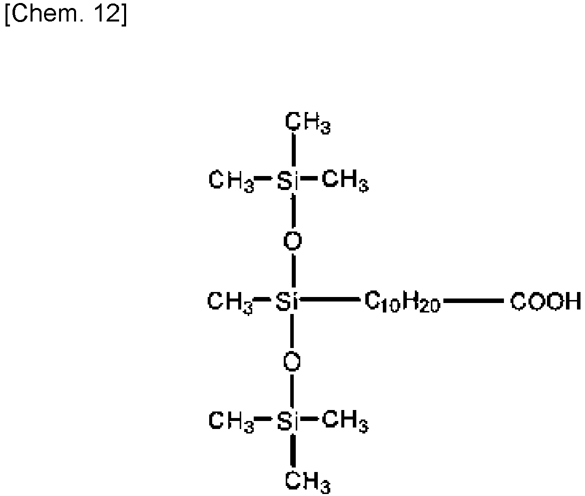

### [Preparation of aqueous dispersion: Examples 1 and 2, Comparative Examples 1 and 2]

The components shown in Table 1 below were put into a mayonnaise bottle, and 160 g of zirconia beads (YTZ ball, ϕ0.8 mm) were added thereinto. After dispersing with a paint shaker and separating the beads, aqueous dispersions of Example 1 and 2 and Comparative Example 1 and 2 were obtained. Each aqueous dispersion was evaluated in the following manner and the results are shown in Table 1. Note that the pH of each aqueous dispersion was measured at room temperature using a skin pH meter skin checker MJ-120 (available from Sato Shoji Corporation).

### [Appearance evaluation for aqueous dispersion]

The appearance of the aqueous dispersion was observed immediately after preparation.
O: Dispersion was good, and low-viscosity dispersion was obtained
X: Dispersion was poor and it became creamy

### [Water repellency test]

The aqueous dispersions of Table 1 were applied to a glass slide, thin and stretched, and dried for 1 hour at 40°C. Water droplets were gently dropped on the surface of the coating film after drying with a dropper, and a state of the water droplets formed on the surface was observed.
O: Water droplets were maintained in hemisphere shape
X: Water droplets spread

### [Water resistance test]

The aqueous dispersions of Table 1 were applied to a glass slide, thin and stretched, and dried for 1 hour at 70°C.
A drop of water was gently dropped on the surface of the coating film after drying with a dropper. The strength of the coating film was confirmed by rotating 10 times while pressing the water droplet with a finger wearing a nitrile glove.
O: No change in coating film
X: Coating film was collapsed

### [Sebum resistance test]

The aqueous dispersions of Table 1 were applied to a glass slide, thin and stretched, and dried for 1 hour at 70°C. An artificial sebum (weight ratio of triolein: oleic acid: squalane = 3: 1: 1) was gently dropped on the surface of the coating film after drying with a dropper. The strength of the coating film was confirmed by rotating 10 times while pressing the liquid droplet with a finger wearing a nitrile glove.
O: No change in coating film
X: Coating film dissolved and collapsed

**[Table 1]**

| | Component | **Example 1** | **Example 2** | **Comparative Example 1** | **Comparative Example 2** |
|---|---|---|---|---|---|
| (1) | Carboxylic acid-modified silicone (Compound 1) | 2 | | - | - |
| (2) | Carboxylic acid-modified silicone (Compound 2) | - | 2 | - | - |
| (3) | Polyether-modified silicone (Note 1) | - | - | 2 | - |
| (4) | Isostearic acid (Note 2) | - | - | - | 2 |
| (5) | Hydrophobic titanium oxide (Note 3) | 12 | 12 | 12 | 12 |
| (6) | Aminomethyl Propanol | 0.57 | 0.48 | - | 0.69 |
| (7) | Ion exchanged water | 16 | 16 | 16 | 16 |
| (8) | 1,3-butanediol | 10 | 10 | 10 | 10 |
| Evaluation results | | | | | |
| pH | | 8.9 | 9.0 | 6.0 | 9.0 |
| Appearance of aqueous dispersion | | ○ | ○ | × | ○ |
| Water repellency test | | ○ | ○ | - | × |
| Water resistance test | | ○ | × | - | × |
| Sebum resistance test | | ○ | × | - | × |

| | | | | | |
|---|---|---|---|---|---|
| (Note 1) PEG-12 dimethicone (Note 2) Trade name: Isostearic acid EX (available from Kokyu Alcohol Kogyo Co., Ltd.) (Note 3) Trade name: MTY-02 (available from Tayca Corporation) | | | | | |

From the evaluation results of Examples 1 and 2, and Comparative Examples 3 and 4, Examples 1 and 2 containing the carboxylic acid-modified silicone were excellent in dispersing the hydrophobic powder and also in water repellency. In Comparative Example 1 containing polyether-modified silicone, dispersibility of the hydrophobic powder was poor, and it was not possible to obtain an aqueous dispersion with low viscosity. In Comparative Example 2 containing isostearic acid, the strength of the coating film was inferior in water repellency, water resistance, and sebum resistance test. Comparing Examples 1 and 2, Example 1, containing the carboxylic acid-modified silicone (compound 1), was excellent in the water resistance and sebum resistance, and the aqueous dispersion did not change in viscosity even after 2 months or more, and maintained good appearance and dispersion stability.

### [Transparency]

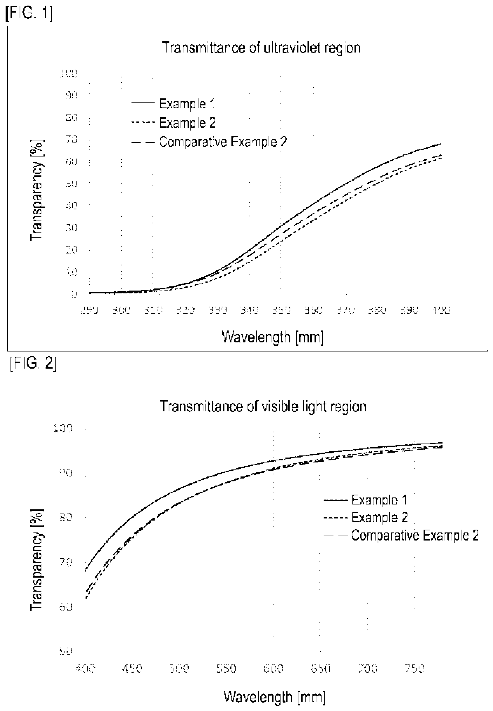

50 µL of the aqueous dispersions obtained in Examples 1 and 2 and Comparative Example 2 were weighed, and 5 ml of ion-exchanged water was added to prepare a solution A. Furthermore, 10 mL of ion-exchanged water was added to 100 µL of solution A to form a solution B, and the ultraviolet light and visible light transmittance of the solution B were measured. The results are shown in FIGS. 1 and 2.

It can be seen from FIG. 1 and FIG. 2 that the dispersion of the present invention is uniformly dispersed, so that the ultraviolet light blocking properties are high and the transmittance in the visible light region is high.

### [Example 3: Preparation example of aqueous dispersion]

3.2 g of carboxylic acid-modified silicone (Compound 1), 20 g of hydrophobic titanium oxide (same as in the aforementioned Note 3), 0.91 g of aminomethyl propanol, 11.2 g of ion-exchanged water, and 5.6 g of 1,3-butanediol were put into a mayonnaise bottle, and 160 g of zirconia beads (YTZ ball, ϕ0.8 mm) were further added thereinto. When dispersed with a paint shaker, it was possible to obtain a uniform aqueous dispersion. As compared with Example 1, the aqueous dispersion of Example 3 has a high content of hydrophobic titanium oxide (50% by mass when the total amount excluding the neutralizing agent is 100% by mass), but has good fluidity and the aqueous dispersion did not change in viscosity, and good appearance and dispersion stability were maintained even after 2 months or more.

### [Example 3-2: Preparation example of aqueous dispersion]

2.0 g of carboxylic acid-modified silicone (Compound 1), 12 g of hydrophobic titanium oxide (trade name: STR-100A-LP; available from Sakai Chemical Industry Co., Ltd.), 0.57 g of aminomethyl propanol, 16 g of ion-exchanged water, and 10 g of ethyl alcohol (= ethanol) were put into a mayonnaise bottle, and 160 g of zirconia beads (YTZ ball, ϕ0.8 mm) were further added thereinto. When dispersed with a paint shaker, it was possible to obtain a stable aqueous dispersion having uniform fluidity at room temperature.

### [Example 4: Preparation example of aqueous dispersion]

2 g of carboxylic acid-modified silicone (Compound 1), 12 g of hydrophobic titanium oxide (same as in the aforementioned Note 3), 0.57 g of aminomethyl propanol, and 26 g of ion-exchanged water were put into a mayonnaise bottle, and 160 g of zirconia beads (YTZ ball, ϕ0.8 mm) were further added thereinto. When dispersed with a paint shaker, it was possible to obtain a uniform aqueous dispersion. That is, an aqueous dispersion can be obtained even in the absence of a polyhydric alcohol corresponding to component (E) in the present invention.

### [Example 5: Preparation example of aqueous dispersion]

2 g of carboxylic acid-modified silicone (Compound 1), 12 g of hydrophobic titanium oxide (Note 4), 0.57 g of aminomethyl propanol, 16 g of ion-exchanged water, and 10 g of 1,3-butanediol were put into a mayonnaise bottle, and 160 g of zirconia beads (YTZ ball, ϕ0.8 mm) were further added thereinto. When dispersed with a paint shaker, it was possible to obtain a uniform aqueous dispersion. Moreover, the aqueous dispersion did not change in viscosity even after 2 months or more, and maintained a good appearance and dispersion stability.
(Note 4) Trade name: FINEX-30S-LPT (available from Sakai Chemical Industry Co., Ltd.)

### [Example 5-2: Preparation example of aqueous dispersion]

2 g of carboxylic acid-modified silicone (Compound 1), 12 g of hydrophobic titanium oxide (Note 5), 0.57 g of aminomethyl propanol, 16 g of ion-exchanged water, and 10 g of 1,3-butanediol were put into a mayonnaise bottle, and 160 g of zirconia beads (YTZ ball, ϕ0.8 mm) were further added thereinto. When dispersed with a paint shaker, it was possible to obtain a uniform aqueous dispersion. Moreover, the aqueous dispersion did not change in viscosity even after 2 months or more, and maintained a good appearance and dispersion stability.
(Note 5) Trade name: FINEX-30-OTS (available from Sakai Chemical Industry Co., Ltd.)

### [Example 6: Preparation example of aqueous dispersion]

3.6 g of carboxylic acid-modified silicone (Compound 1), 22 g of hydrophobic titanium oxide (same as in the aforementioned Note 3), 1.03 g of aminomethyl propanol, 8.8 g of ion-exchanged water, and 5.6 g of 1,3-butanediol were put into a mayonnaise bottle, and 160 g of zirconia beads (YTZ ball, ϕ0.8 mm) were further added thereinto. When dispersed with a paint shaker, it was possible to obtain a uniform aqueous dispersion. As compared with Example 1, the aqueous dispersion of Example 6 has a high content of hydrophobic titanium oxide (55% by mass when the total amount excluding the neutralizing agent is 100% by mass), has fluidity at room temperature.

### [Example 7: Preparation example of aqueous dispersion]

4.0 g of carboxylic acid-modified silicone (Compound 1), 24 g of hydrophobic zinc oxide (same as in the aforementioned Note 4), 1.14 g of aminomethyl propanol, 7.4 g of ion-exchanged water, and 4.6 g of 1,3-butanediol were put into a mayonnaise bottle, and 160 g of zirconia beads (YTZ ball, ϕ0.8 mm) were further added thereinto. When dispersed with a paint shaker, it was possible to obtain a uniform aqueous dispersion. As compared with Example 1, the aqueous dispersion of Example 7 has a high content of hydrophobic oxide powder (60% by mass when the total amount excluding the neutralizing agent is 100% by mass), has fluidity at room temperature.

### [Example 8: Preparation example of aqueous dispersion]

5 g of carboxylic acid-modified silicone (Compound 1), 25 g of 1,3-butanediol, 0.67 g of aminomethylpropanol, and 30 g of hydrophobic zinc oxide (same as in the aforementioned Note 4) were put into a 100 cc beaker, and stirred lightly with a glass rod. After applying this mixture to three rolls, 40 g of water was added and the mixture was stirred, and thereby it was possible to obtain a uniform aqueous dispersion having fluidity at room temperature.

### [Comparative Example 4: Preparation example of aqueous dispersion]

5 g of carboxylic acid-modified silicone (Compound 1), 25 g of 1,3-butanediol, and 30 g of hydrophobic zinc oxide (same as in the aforementioned Note 4) were put into a 100 cc beaker, and stirred lightly. After applying this mixture to three rolls, 40 g of water was added and stirred to obtain a uniform aqueous dispersion, but the viscosity was higher as compared to Example 8.

### [Example 9: Preparation example of aqueous dispersion]

2.5 g of carboxylic acid-modified silicone (Compound 1), 25 g of 1,3-butanediol, 0.4 g of aminomethylpropanol, and 30 g of a hydrophobic pigment mix (Note 5) were put into a 100 cc beaker, and stirred lightly. After applying this mixture to three rolls, 40 g of water was added and the mixture was stirred, and thereby it was possible to obtain a uniform aqueous dispersion having fluidity at room temperature.
(Note 5) Composition of hydrophobic pigment mix
87.2 parts by weight of SI Titanium CR-50, available from Miyoshi Kasei, Inc.
10.2 parts by weight of SA Yellow-LLXLO available from Miyoshi Kasei, Inc.
2.1 parts by weight of SA RED, available from Miyoshi Kasei, Inc.
0.5 parts by weight of SA black BL-100, available from Miyoshi Kasei, Inc.

### [Comparative Example 5: Preparation example of aqueous dispersion]

2.5 g of PEG-12 dimethicone, 25 g of 1,3-butanediol, and 30 g of a hydrophobic pigment mix (Note 5) were put into a 100 cc beaker, and stirred lightly. After applying this mixture to three rolls, 40 g of water was added and stirred to obtain a uniform aqueous dispersion, but the viscosity was higher as compared to Example 9.

Hereinafter, the production of a cosmetic composition using the aqueous dispersion of the present invention will be specified with reference to Formulation Examples 1 to 5. Note that, the use of the aforementioned compound 1 as carboxylic acid-modified silicone used in the aqueous dispersion of the present invention is suitable, and one that is available as the trade name ES-5800 Formulation Aid (available from Dow Toray Co., Ltd.) can be suitably used.

### [Formulation Example 1: Oil-in-water sunscreen agent]

**[Table 2]**

| Component | | Compounding amount |
|---|---|---|
| A | Silicone emulsifier premix (Note 6) | 10 |
| | Trilaureth-4 phosphate | 0.05 |
| | Phenyl trimethicone | 2 |
| | Caprylyl methicone | 3 |
| | Ethylhexyl methoxycinnamate | 6.7 |
| | Diethylaminohydroxybenzoyl hexyl benzoate | 3.3 |
| B | Carbomer | 0.3 |
| | Purified water | Remaining amount |
| | Na hydroxide | Appropriate amount |
| | Butylene glycol | 3 |
| | Glycerin | 5 |
| | Preservative | Appropriate amount |
| C | Aqueous dispersion (Example 1) | 15 |

| | | |
|---|---|---|
| (Note 6) DOWNSIL™ FB-2540 Emulsifier BLEND (available from Dow Toray Co., Ltd.) | | |

### (Producing Method)

(1) The components of phase A are mixed.
(2) The components of phase B are mixed.
(3) An emulsion is prepared by adding a mixture of components of the phase A to a mixture of components of the phase B in small portions at normal room temperature.
(4) A cosmetic composition is prepared by mixing phase C with the above emulsion.

### [Formulation example 2: Oil-in-water foundation]

**[Table 3]**

| Component | | Compounding amount |
|---|---|---|
| A | PEG-12 dimethicone (Note 7) | 4 |
| | Lauryl PEG/PPG-18/18 Methicone (Note 8) | 0.5 |
| | Stearyl dimethicone (Note 9) | 3 |
| | (Trimethylsiloxysilicate/dimethiconol) Cross polymer (Note 10) | 2 |
| | Caprylyl methicone | 6.5 |
| | Ethylhexyl methoxycinnamate | 5 |
| | (Dimethicone/Vinyl Dimethicone) Crosspolymer, Silica (Note 11) | 4 |
| B | Carbomer | 0.6 |
| | Glycerin | 3 |
| | Purified water | Remaining amount |
| | Na hydroxide | Appropriate amount |
| | Preservative | Appropriate amount |
| C | Aqueous dispersion (Example 9) | 33.3 |

| | | |
|---|---|---|
| (Note 7) DOWNSIL™ ES-5373 FORMULATION AID (available from Dow Toray Co., Ltd.) (Note 8) DOWSIL™ 5200 FORMULATION AID (available from Dow Toray Co., Ltd.) (Note 9) DOWSIL™ 2503 COSMETIC WAX (available from Dow Toray Co., Ltd.) (Note 10) DOWSIL™ FC-5002 IDD RESIN GUM (available from Dow Toray Co., Ltd.) (Note 11) DOWSIL™ 9701 COSMETIC POWDER (available from Dow Toray Co., Ltd.) | | |

### (Producing Method)

(1) The components of phase A are mixed.
(2) The components of phase B are mixed.
(3) An emulsion is prepared by adding a mixture of components of the phase A to a mixture of components of the phase B in small portions at normal room temperature.
(4) A cosmetic composition is prepared by mixing phase C with the above emulsion.

### [Formulation Example 3: Oil-in-water sunscreen agent]

**[Table 4]**

| Component | | Compounding amount |
|---|---|---|
| A | Carboxylic acid-modified silicone (Compound 1) | 1.5 |
| | Glycerin | 5 |
| | 50% aqueous solution of sodium hydroxide | 0.5 |
| | Tri(capryl/capric acid) glyceryl | 4 |
| | Ethylhexyl methoxycinnamate | 7.5 |
| | Diethylaminohydroxybenzoyl hexyl benzoate | 2 |
| B | Acrylate copolymer (Note 12) | 3 |
| | Purified water | Remaining amount |
| | Triethanolamine | Appropriate amount |
| | Phenoxyethanol | 0.9 |
| C | Aqueous dispersion (Example 5) | 13.3 |

| | | |
|---|---|---|
| (Note 12) ACULYN™ 33A RHEOLOGY MODIFIER (available from The Dow Chemical Company) | | |

### (Producing Method)

(1) The components of phase A are mixed.
(2) The components of phase B are mixed.
(3) An emulsion is prepared by adding a mixture of components of the phase A to a mixture of components of the phase B in small portions at normal room temperature.
(4) A cosmetic composition is prepared by mixing phase C with the above emulsion.

### [Formulation Example 4: Oil-in-water sunscreen agent]

**[Table 5]**

| Component | | Compounding amount |
|---|---|---|
| A | Polysorbate 80 | 1 |
| | Mineral oil | 10 |
| | Triethylhexanoin | 5 |
| | Caprylyl methicone | 10 |
| | Cyclopentasiloxane | 5 |
| | Ethylhexyl methoxycinnamate | 7.5 |
| | Diethylaminohydroxybenzoyl hexyl benzoate | 2.5 |
| B | Carbomer | 0.3 |
| | Purified water | Remaining amount |
| | Na hydroxide | Appropriate amount |
| | Butylene glycol | 2 |
| | Glycerin | 2 |
| | Phenoxyethanol | 0.9 |
| C | Aqueous dispersion (Example 5) | 13.3 |
| D | Silicone-modified acrylic emulsion (Note 13) | 3 |

| | | |
|---|---|---|
| (Note 13) FA-4103 SILICONE ACRYLATE EMULSION (available from Dow Toray Co., Ltd.) | | |

### (Producing Method)

(1) The components of phase A are mixed, and warmed to 70°C.
(2) The components of phase B are mixed, and warmed to 70°C.
(3) An emulsion is prepared by adding a mixture of components of the phase A to a mixture of components of the phase B in small portions.
(4) The emulsion is cooled to near room temperature while stirring.
(5) The emulsion is added in the order of the C phase and the D phase to prepare a cosmetic composition.

### [Formulation example 5: Oil-in-water foundation]

**[Table 6]**

| Component | | Compounding amount |
|---|---|---|
| A | Carboxylic acid-modified silicone (Synthesis Example 1) | 1.5 |
| | Glycerin | 5 |
| | 50% KOH aqueous solution | 0.5 |
| | Caprylic/capric triglyceride | 4 |
| | Cyclopentasiloxane | 3 |
| | Ethylhexyl methoxycinnamate | 3 |
| B | Acrylate copolymer (Note 12) | 3 |
| | Water | Residual |
| | Triethanolamine | Appropriate amount |
| | Phenoxyethanol | 0.9 |
| C D | Aqueous dispersion (Example 9) | 33.3 |
| | (Divinyl Dimethicone/Dimethicone) Copolymer (Note 14) | 4.2 |

| | | |
|---|---|---|
| (Note 14) DOWSIL™ HMW2220 NON-IONIC EMULSION (available from Dow Toray Co., Ltd.) | | |

### (Producing Method)

(1) The components of phase A are mixed.
(2) The components of phase B are mixed.
(3) An emulsion is prepared by adding a mixture of components of the phase A to a mixture of components of the phase B in small portions at normal room temperature.
(4) The phase C is mixed with the above emulsion.
(5) A cosmetic composition is prepared by mixing phase D with the above emulsion.

### [Formulation example 6: Oil-in-water foundation]

**[Table 7]**

| Component | | Compounding amount |
|---|---|---|
| A | Stearic acid | 1 |
| | Polysorbate 80 | 1.2 |
| | Sorbitan sesquioleate | 0.2 |
| | Glyceryl stearate | 1.5 |
| | Behenyl alcohol | 2.5 |
| | Dimethicone | 11 |
| | Squalene | 3 |
| | Isotridecyl isononanoate | 3 |
| | Caprylic/capric triglyceride | 3 |
| B | Carbomer | 0.1 |
| | Purified water | Remaining amount |
| | Na hydroxide | Appropriate amount |
| | Butylene glycol | 6.3 |
| | Phenoxyethanol | 0.9 |
| C | Aqueous dispersion (Example 9) | 33.3 |
| D | Acrylate copolymer (Note 15) | 3 |

| | | |
|---|---|---|
| (Note 15) EPITEX™ 66 polymer (available from The Dow Chemical Company) | | |

### (Producing Method)

(1) The components of phase A are mixed, and warmed to 70°C.
(2) The components of phase B are mixed, and warmed to 70°C.
(3) An emulsion is prepared by adding a mixture of components of the phase A to a mixture of components of the phase B in small portions.
(4) The emulsion is cooled to near room temperature while stirring.
(5) The emulsion is added in the order of the C phase and the D phase to prepare a cosmetic composition.

### [Formulation Example 7: sunscreen cream]

**[Table 8]**

| Phase | Display Name | Product name/supplier | % Wt |
|---|---|---|---|
| A | Ethylhexyl methoxycinnamate | Ubiqual MC-80 N/BASF | 1.5 |
| | Ethylhexyl salicylate | PARSOL EHS/DSM Nutritional Products Japan | 5 |
| | Homosalate | Eusolex (registered mark) HMS/Merck | 6 |
| | Butyl octyl salicylate | | 5 |
| | t-butylmethoxydibenzoylmethane | PARSOL/ 1789/DSM Nutritional Products Japan | 1.5 |
| B | Cetearyl alcohol | Cetearyl alcohol/higher alcohol industry | 0.5 |
| | (VP/eicosene) copolymer | ANTARON V220/Ashland Japan. | 2 |
| | Glyceryl stearate | | 1.9 |
| | Stearic acid PEG-100 | EMALEX 8100/NIHON EMULSION Co., Ltd. | 1.95 |
| | Cetyl phosphate K, hydrogenated palm fatty acid glyceriz | | 1.4 |
| C | Water | | Residual |
| | EDTA-2Na | | 0.05 |
| | Xanthan gum | | 0.3 |
| | Preservative | | Appropriate amount |
| | Glycerin | | 3 |
| D | (Acrylamide/acryloyldimethyltaurine Na) copolymer, isohexadecane, polysorbate 80 | SIMULGEL EG/SEPPIC | 1 |
| E | Water dispersion (Example 4) | | 15 |
| | Water dispersion (Example 5) | | 15 |

### (Producing Method)

Phase A is mixed.
Phase B is mixed.
Phase A is added to Phase B and mixture is warmed to 85°C.
Phase C is warmed to 80°C.
Phases A and B are slowly added while stirring Phase C.
ABC phases are cooled to 50°C while stirring.
Phase D is added while stirring ABC phases and mixture is cooled to room temperature.

### [Formulation Example 8: sunscreen cream]

**[Table 9]**

| Phase | Product name abbreviation | | Wt% |
|---|---|---|---|
| A | Isostearic acid EX, available from Kokyu Alcohol Kogyo Co., Ltd. | Isostearic acid | 1.5 |
| | | Glycerin | 5 |
| | | Hydroxide K | 0.25 |
| | | Water | 0.25 |
| B | | Tri(capryl/capric acid) glyceryl | 2 |
| | Uvinul MC 80, available from BASF | Ethylhexyl methoxycinnamate | 7.5 |
| | Uvinul A plus, available from BASF | Diethylaminohydroxybenzoyl hexyl benzoate | 2 |
| C | CEKOL available from Sansho Co., Ltd. | Cellulose gum | 0.3 |
| | | Glycerin | 3 |
| | | Water | Residual |
| | | Phenoxyethanol | 0.9 |
| D | Aqueous dispersion of Example 4 | | 13.3 |
| | Water dispersion of Example 5 | | 13.3 |

### (Producing Method)

(1) Phase A is mixed.
(2) Phase B is mixed.
(3) Phase C is mixed.
(4) Phase B is slowly added while stirring Phase A.
(5) Phases A and B are added to Phase C.
(6) Phase D is added and mixed into Phases ABC.

## Claims

1. An aqueous dispersion composition comprising:
at least (A) carboxylic acid-modified silicone in a liquid form at 50°C;
(B) a hydrophobic powder;
(C) a basic substance;
(D) water; and
optionally, (E) one or more alcohols selected from polyhydric alcohols and ethyl alcohols; wherein:
80% by mass or more of the total composition is formed of the components (A) to (E),
the component (B) is contained in a range of 10% to 70% by mass of the total composition, and
the component (A) is contained in a range of 5 to 30 parts by mass with respect to 100 parts by mass of the component (B).

2. The aqueous dispersion composition according to claim 1, wherein 90% by mass or more of the total composition is formed of the components (A) to (E).

3. The aqueous dispersion composition according to claim 1 or 2, wherein a content of the component (D) is in a range of 10% to 80% by mass with respect to the total composition.

4. The aqueous dispersion composition according to any one of claims 1 to 3, which is substantially free of an oil agent.

5. The aqueous dispersion composition according to any one of claims 1 to 4, wherein the (A) carboxylic acid-modified silicone is represented by the following structural formula (1): 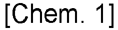 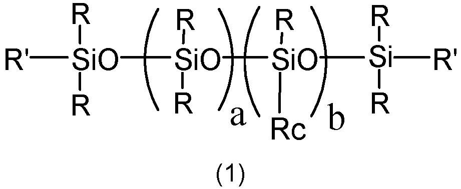 (wherein:
Rc represents a carboxyl group-containing organic group represented by a general formula: -R¹-(OR²)p-(O)w-R³-COOH, (R¹ represents a linear or branched alkylene group having 2 to 22 carbon atoms, R² represents a linear or branched alkylene group having 2 to 4 carbon atoms, R³ represents a bond (-) or a linear or branched alkylene group having 1 to 22 carbon atoms, p represents a number from 0 to 200, and w represents a number of 0 or 1),
R represents the same or different alkyl or alkoxy group, having 1 to 22 carbon atoms, or phenyl group,
R' is Rc or R, and
a and b are 0 or positive numbers, respectively, a + b is a number in a range of 0 to 30, and when b is 0, at least one of R' is Rc).

6. The aqueous dispersion composition according to claim 5, wherein in the structural formula (1), the (A) carboxylic acid-modified silicone is carboxylic acid-modified silicone which is in a liquid form at room temperature (25°C) in which a + b is a number in a range of 2 to 20, and a/b is in a range of 0.3 to 3.0.

7. The aqueous dispersion composition according to any one of claims 1 to 6, wherein a pH of the total composition is in a range of 6.5 to 14.0.

8. The aqueous dispersion composition according to any one of claims 1 to 7, which is a material for a cosmetic composition.

9. A method of producing a cosmetic composition, comprising:
using the aqueous dispersion composition described in any one of claims 1 to 7.
